# EUROPÄISCHE PATENTSCHRIFT

(11) **EP 3 493 665 B1**
(45) Veröffentlichungstag und Bekanntmachung des Hinweises auf die Patenterteilung: **23.08.2023**
(21) Anmeldenummer: 17749363.2
(22) Anmeldetag: 31.07.2017
(51) Int. Cl.: A01H 4/00, A01G 9/08

(54) **VORRICHTUNG UND VERFAHREN ZUM VERMEHREN VON PFLANZEN**
DEVICE AND METHOD FOR PROPAGATING PLANTS
DISPOSITIF ET PROCÉDÉ DE REPRODUCTION DE PLANTES

(30) Priorität: 03.08.2016 DE 102016009352; 05.09.2016 DE 102016010618
(43) Veröffentlichungstag der Anmeldung: 12.06.2019
(73) Patentinhaber: RoBoTec PTC GmbH, 28357 Bremen (DE)
(72) Erfinder: VON RUNDSTEDT, Stephan, 28357 Bremen (DE); VON RUNDSTEDT, Friederike, 28357 Bremen (DE)
(74) Vertreter: Möller, Friedrich
(86) Internationale Anmeldenummer: PCT/EP2017/000922
(87) Internationale Veröffentlichungsnummer: WO 2018/024369

(56) Entgegenhaltungen:
- WO-A1-92/03913

## Beschreibung

Die Erfindung betrifft ein Verfahren zum Vermehren von Pflanzen gemäß dem Anspruch 1. Des Weiteren betrifft die Erfindung eine Vorrichtung zum Vermehren von Pflanzen gemäß dem Anspruch 7.

Zier- und Nutzpflanzen werden zunehmend für industrielle Anwendungen benötigt. Dabei kommt es insbesondere auf definierte Eigenschaften dieser Pflanzen wie das optische Erscheinungsbild, einem hohen Ölgehalt, bestimmte Resistenzen oder dergleichen an. Um der erhöhten Nachfrage nachzukommen, werden Setzlinge dieser Pflanzen in Laboratorien unter sterilen Bedingungen als Klone über Gewebekulturen in hoher Stückzahl produziert. Diese in vitro Vermehrung der Pflanzen, insbesondere die Vermehrung von "maßgeschneiderten" Pflanzensetzlingen, erfolgt händisch. Das heißt, dass die einzelnen Pflanzensetzlinge von einer Person aufgenommen und für die Vermehrung entsprechend zugeschnitten werden müssen. Die so erzeugten Klone der Pflanzen werden sodann einem Nährboden zugeführt, damit die Klone weiter wachsen und sich durch Sprossbildung vermehren.

Dieses in vitro Verfahren ist sehr personalintensiv und somit mit sehr hohen Kosten verbunden. Da die Vermehrung von Pflanzen mit definierten Eigenschaften allerdings zu einer Schlüsselindustrie der Biotechnologie gehört, müssen für den Ausbau dieser Technologie die Kosten dramatisch gesenkt werden. Außerdem besteht durch die händische Bearbeitung an offenen Werkbänken eine laufende Kontaminationsgefahr mit Keimen, welche zum Verlust der gesamten Produktion führen kann. Teilautomatisierte in vitro Verfahren, wie beispielsweise in WO 92/03913 beschrieben, bieten keine Alternative für die händische Bearbeitung, da die notwendige Schnittgenauigkeit nicht erreichbar ist.

Der Erfindung liegt daher die Aufgabe zugrunde, ein Verfahren sowie eine Vorrichtung zum Vermehren von Pflanzen zu schaffen, durch welche die Kosten der Produktion von Pflanzen gesenkt werden können.

Eine Lösung dieser Aufgabe wird durch die Maßnahmen des Anspruchs 1 beschrieben. Demnach ist ein Verfahren vorgesehen, bei dem die zu vermehrenden Pflanzen automatisiert durch einen ersten Greifer ergriffen und vereinzelt werden, die einzelnen Pflanzen, an dem ersten Greifer hängend, gezielt in mehrere Klone zerschnitten und die einzelnen Klone automatisiert durch einen zweiten Greifer für die weitere Verarbeitung abtransportiert werden. Durch diese Vollautomatisierung des Verfahrens zum Vermehren der Pflanzen kann nahezu vollständig auf den Einsatz von kostenintensivem Personal verzichtet werden. Durch diese Automatisierung des Verfahrens wird es somit möglich, die Kosten für die Produktion von Pflanzen massiv zu reduzieren. Darüber hinaus wird durch dieses Verfahren die Kontaminationsgefahr minimiert. Weiter ist es vorgesehen, dass durch eine zweite Bilderkennungseinrichtung Schnittlinien an der am ersten Greifer hängenden Pflanze ermittelt werden, entlang derer die Pflanze automatisiert durch ein Messer, einen Laserstrahl, einen Wasserstrahl oder einen Plasmastrahl in Klone der Pflanze zerschnitten wird. Dabei kann die Schnittlinie durch die Pflanze den vorbestimmten Vorgaben entsprechend von der Bilderkennungseinrichtung bestimmt werden. Vor der Durchführung des Vermehrungsprozesses können bereits Kriterien festgelegt und an die Steuereinrichtung der Bilderkennungseinrichtung übermittelt werden, nach denen der Schnitt zu erfolgen hat, damit die Pflanze problemlos nach dem Zerschneiden weiter wachsen kann. Das Zerschneiden der Pflanze mittels Laserstrahl stellt eine kontaktfreie und genaue Methode zum Teilen einer Pflanze dar.

Dieses Verfahren ist sowohl auf Nutz- als auch auf Zierpflanzen anwendbar. Insbesondere kann es erfindungsgemäß vorgesehen sein, dass die zu vermehrenden Pflanzen in einem zunächst verschlossenen, insbesondere markierten, Behälter in einem geschlossenen, sterilen Raum eingeführt werden und die Behälter in dem Raum automatisiert, insbesondere durch mindestens einen Saugnapf, geöffnet werden. Um zu vermeiden, dass sich Keime, die sich negativ auf das Wachstum der Pflanzen auswirken können, auf weitere Pflanzen ausbreiten, erfolgt das gesamte Verfahren zum Vermehren von Pflanzen in einem geschlossenen bzw. sterilen Raum. Bei diesem zellenartigen Raum kann es sich beispielsweise auch um einen Reinraum handeln. Um die Atmosphäre in dem geschlossenen Raum möglichst steril zu halten, werden die Pflanzensetzlinge bzw. die Pflanzen zunächst in einem geschlossenen Behälter durch beispielsweise eine Schleuse in den Raum eingeführt. Dieser Behälter wird durch eine Einrichtung zum Öffnen des Behälters, wie beispielsweise einem Saugnapf, geöffnet. Der an den Saugnäpfen befestigte Deckel des Behälters kann sodann entsorgt werden. Informationen, die eventuell an dem Deckel positioniert sind und den Inhalt des Behälters beschreiben, können beispielsweise automatisch eingelesen und gespeichert werden und dienen dem weiteren Verfahren als Identifikation der einzelnen Setzlinge. Der geöffnete Behälter wird sodann durch eine entsprechende Vorrichtung weitertransportiert.

Ein weiteres vorteilhaftes Ausführungsbeispiel der vorliegenden Erfindung kann es vorsehen, dass der geöffnete Behälter einer ersten Bilderkennungseinrichtung zugeführt wird und durch die Bilderkennungseinrichtung geeignete Positionen ermittelt werden, an denen die einzelnen Pflanzen durch den ersten Greifer nacheinander erfasst und aus dem Behälter entnommen werden. Die einzelnen sich in dem Behälter befindlichen Pflanzen bzw. Pflanzensetzlinge bilden eine extrem ungleichmäßige, d. h. inhomogene Topographie aus Blättern, Stängeln oder dergleichen. Damit die Pflanzen durch den ersten Greifer gezielt erfasst werden können und nicht etwa ins Leere greifen oder gar eine Position greift, auf der später der Schnitt durchgeführt werden soll, wird zunächst die gesamte Topographie der Pflanzen, d. h. die Gesamtheit der Blätter, die in dem Behälter nach oben ragen, aufgenommen und durch eine Steuereinrichtung bearbeitet. Diese Steuereinrichtung bestimmt dann per Bilderkennung welche Position an den Pflanzen sich besonders gut eignet für die Aufnahme der Pflanze durch den Greifer. Wenn so eine Position ermittelt wurde, wird der, insbesondere zangenartige, Greifer durch einen Roboterarm an die entsprechende Position geführt und der Pflanzensetzling aus dem Behälter genommen. Dabei werden die einzelnen Behälter durch einen Unterdruck auf dem Boden fixiert. Die so ergriffene Pflanze wird sodann durch den Roboterarm einer weiteren Station für die Vermehrung der Pflanzen zugeführt.

Erfindungsgemäß kann es vorgesehen sein, dass die Klone, vorzugsweise auf einem Förderband, zu einer dritten Bilderkennungseinrichtung transportiert werden, Positionen ermittelt werden, an denen die einzelnen Klone durch einen zweiten Greifer ergriffen und in einem Behälter, vorzugsweise gemäß einem programmierten Setzmuster in dem Behälter, abgesetzt werden. Die von der an dem ersten Greifer gehaltenen Pflanze abgeschnittenen Klone fallen schwerkraftbedingt auf das Förderband, welches genau unterhalb der Einrichtung zum Schneiden der Pflanze angeordnet ist. Durch dieses Förderband vorangetrieben werden die Klone sodann in einen Bereich geführt, dem die dritte Bilderkennungseinrichtung zugeordnet ist. Durch diese Bilderkennungseinrichtung wird eine Position an den einzelnen Klonen bestimmt, die als besonders geeignet erscheint, um den Klon durch einen zweiten Greifer zu ergreifen und in einem vorbereiteten Behälter abzusetzen. Bei dem Absetzen kann der Greifer einer vorbestimmten Matrix folgen, damit die Pflanzen in einer möglichst hohen Dichte in dem Behälter positioniert werden, ohne sich gegenseitig zu stören.

Ein weiteres Ausführungsbeispiel der vorliegenden Erfindung kann es vorsehen, dass der ein Nährmedium enthaltene Behälter vor der Aufnahme der Klone in dem sterilen Raum geöffnet und nach der Aufnahme der Klone in dem Raum wieder geschlossen wird, bevor der Behälter aus dem Raum abtransportiert wird. Genau wie die Behälter, in denen die Pflanzen dem Raum zugeführt wurden, werden auch die Behälter, in denen die Klone abgeführt werden, durch eine auf dem Boden der Behälter wirkende Saugkraft fixiert. Die Behälter werden dem Raum mit einem gekennzeichneten Deckel zugeführt. Die Kennzeichnung des Deckels enthält Informationen über den Nährboden. Nach dem Abnehmen des Deckels durch beispielsweise einen Saugnapf und dem Zufügen der einzelnen Klone in den Behälter, wird der Behälter wieder mit dem gleichen Deckel verschlossen. Bei Entnahme der frisch belegten Behälter werden diese mit Informationen der aufgenommenen Klone versehen. Auf diese Weise kann beispielsweise eine Kreuzkontaminierung verschiedener Behälter vermieden werden. Darüber hinaus bleibt eine lückenlose Dokumentation der Behälter und der Klone über den gesamten Prozess der Vermehrung bestehen. Die den Nährboden enthaltenen Behälter werden über eine Rutsche oder ein Förderband dem Raum zugeführt. Gleichermaßen können die mit den Klonen befüllten Behälter den Raum beispielsweise über eine Schleuse wieder verlassen.

Die Erfindung kann es weiter vorsehen, dass der Raum und/oder die Greifer regelmäßig, insbesondere nach dem Durchgang einer zu vermehrenden Pflanzencharge, sterilisiert werden. Diese Sterilisation dient dem Abtöten von Keimen, die beispielsweise durch eine kontaminierte Pflanzencharge in dem Raum gelangt ist. Zur Dekontaminierung dient beispielsweise eine UV-Bestrahlung durch in dem Raum angeordnete entsprechende Leuchtmittel, durch Erhitzung oder durch Begasung des Raumes mit beispielsweise Wasserstoffperoxid.

Eine Vorrichtung zur Lösung der eingangs genannten Aufgabe weist die Merkmale des Anspruchs 7 auf. Demnach ist eine Vorrichtung vorgesehen mit mindestens einem ersten Greifer zum gezielten Ergreifen und Vereinzeln einer zu vermehrenden Pflanze, mit mindestens einer Einrichtung zum automatisierten Zuschneiden der Pflanze, in mehrere Klone, und mit mindestens einem zweiten Greifer zum automatisierten Abtransport einzelner Klone, wobei die Greifer und die mindestens eine Einrichtung zum Zerschneiden in einem Raum angeordnet sind. Durch diese Automatisierung der Vorrichtung zum Vermehren der Pflanzen ist ein händisches Zutun einer Bedienperson nicht mehr erforderlich. Durch die beanspruchte Vorrichtung erfolgt die gesamte in vitro Vermehrung der Pflanzen vollautomatisiert. Sowohl die Entnahme der Pflanzensetzlinge aus einem Behälter, die Vereinzelung der Pflanzen, das Klonieren sowie das Zuführen der Klone in ein Nährmedium für den weiteren Wachstumsprozess werden durch ein Steuersystem automatisch gesteuert. Dadurch lassen sich die Produktionskosten von industriell hergestellten Pflanzen reduzieren. Es ist außerdem vorgesehen, dass die Einrichtung zum automatisierten Zerschneiden der Pflanze ein Laser, ein Plasmagenerator, ein Messer, eine Zange oder ein Wasserschneider ist, der eine zweite Bilderkennungseinrichtung zugeordnet ist zur Ermittlung geeigneter Schnittlinien, wobei der Einrichtung zum automatisierten Zerschneiden der Pflanze ein Förderband zum Abfördern der Klone zugeordnet ist.

Die Erfindung kann es bevorzugt vorsehen, dass der Raum ein steriler Reinraum ist mit mehreren Zugängen, insbesondere Schleusen, für das Einschleusen und Ausschleusen von Behältern mit den zu vermehrenden Pflanzen und Klonen, wobei dem Raum, insbesondere den Zugängen des Raumes, mindestens zwei Förderorgane, vorzugsweise Drehteller, für die Behälter zugeordnet sind, mit denen die Behälter automatisiert in vorbestimmte Positionen verfahrbar sind. Behälter, die durch die Schleusen in den Raum zur Vermehrung der Pflanzen gelangen, werden direkt von Aufnahmen in den Drehtellern aufgenommen und durch eine rotierende Bewegung der Förderorgane von selbigen abtransportiert. Durch das Drehen der Förderorgane bzw. der Drehteller werden die Behälter mit den Pflanzen entlang den verschiedenen Stationen zum Vermehren von Pflanzen geführt und als leere Behälter am Ende der Rotation durch eine Schleuse aus dem Raum ausgeschleust.

Der Raum kann an einer Oberseite ein Belüftungssystem aufweisen, durch welches eine sterile Atmosphäre in dem Raum erhalten werden kann. Durch zusätzliche Sterilisatoren wie UV-Lampen, Begasungssysteme oder dergleichen lässt sich der Raum nach jedem Vermehrungsprozess einer bestimmten Pflanze sterilisieren. Zusätzlich kann der Raum beispielsweise Sensoren aufweisen, mit denen die Sterilität des Raumes nachweisbar bzw. dokumentierbar ist. Dazu kann beispielsweise auch ein mit Nährlösung gefüllter Behälter dienen, der in regelmäßigen Abständen auf Bewuchs kontrolliert werden kann.

Insbesondere kann es weiter vorgesehen sein, dass den Förderorganen mindestens eine Einrichtung, insbesondere mindestens ein Saugnapf, zum automatisierten Öffnen und/oder Schließen der Behälter zugeordnet ist. Beim Einführen der Behälter in den Raum werden die Deckel der Behälter durch einen Saugnapf abgenommen, während der Behälterkörper selbst durch eine entsprechende Gegenkraft, die beispielsweise auch durch einen Saugnapf erzeugt werden kann, an einem Boden des Förderorgans festgehalten wird. Der Saugnapf ist derart beweglich ausgebildet, dass der Deckel in einem Entsorgungsschacht entsorgt werden kann. Behältern, die zur Aufnahme der Klone dienen, ist ein Saugnapf zur Abnahme des Deckels zugeordnet. Dieser abgenommene Deckel wird sodann an einen weiteren Saugnapf übergeben, welcher wiederum den Deckel nach Befüllung des Behälters mit den Klonen schließt.

Ein weiteres vorteilhaftes Ausführungsbeispiel der vorliegenden Erfindung kann es vorsehen, dass mindestens einem Förderorgan, vorzugsweise beiden Förderorganen und/oder einem Förderband, jeweils eine Bilderkennungseinrichtung zugeordnet ist zur Ermittlung einer geeigneten Position, an der eine Pflanze und/oder ein Klon durch den ersten und/oder zweiten Greifer, insbesondere durch eine an einem mehrachsigen Roboterarm angeordnete Pinzette, automatisiert greifbar ist. Bei dieser Bilderkennungseinrichtung kann es sich beispielsweise um eine CCD-Kamera handeln, die mit einem Steuersystem gekoppelt ist. Durch dieses Steuersystem werden die aufgenommenen Formen analysiert und mit einem Datensystem abgeglichen. Durch dieses Abgleichen können bevorzugte Positionen an den Pflanzen ermittelt werden, die sich besonders gut eignen zum Ergreifen der Pflanzen durch den Greifer. Die Steuerung für die Bilderkennungseinrichtungen, für die Greifer sowie für die Roboterarme befindet sich in einem Basiselement der Vorrichtung. Die den Greifer haltenden Roboterarme sind derart ausgelegt, dass sie sich in alle Raumrichtungen bewegen können und somit eine Übergabe der Pflanzen von einer Position in eine andere Position möglich ist. Der Greifer besteht im Wesentlichen aus einer Pinzette, welche durch einen Stellmotor gezielt geöffnet und geschlossen werden kann. An den Spitzen der Pinzette befinden sich zwei Verbreiterungen, um die Aufnahme der Pflanzen besonders schonend zu gestalten.

Auch bei der zweiten Bilderkennungseinrichtung handelt es sich vorzugsweise um eine CCD-Kamera, die mit einer Steuereinrichtung verbunden ist. Der Laser, bei dem es sich beispielsweise um einen CO2-Laser handeln kann, ist derart verfahrbar gelagert, dass er den von der Bilderkennungseinrichtung berechneten Schnittlinien exakt folgen kann und somit aus der Pflanze mehrere Klone schneiden kann.

Ein bevorzugtes Ausführungsbeispiel der Erfindung wird im Folgenden anhand der Zeichnung näher beschrieben. In dieser zeigen:
- Fig. 1: eine schematisierte Darstellung einer Vorrichtung in einem ersten Verfahrensschritt,
- Fig. 2: eine schematisierte Darstellung der Vorrichtung in einem zweiten Verfahrensschritt, und
- Fig. 3: eine schematisierte Darstellung der Vorrichtung gemäß einem dritten Verfahrensschritt.

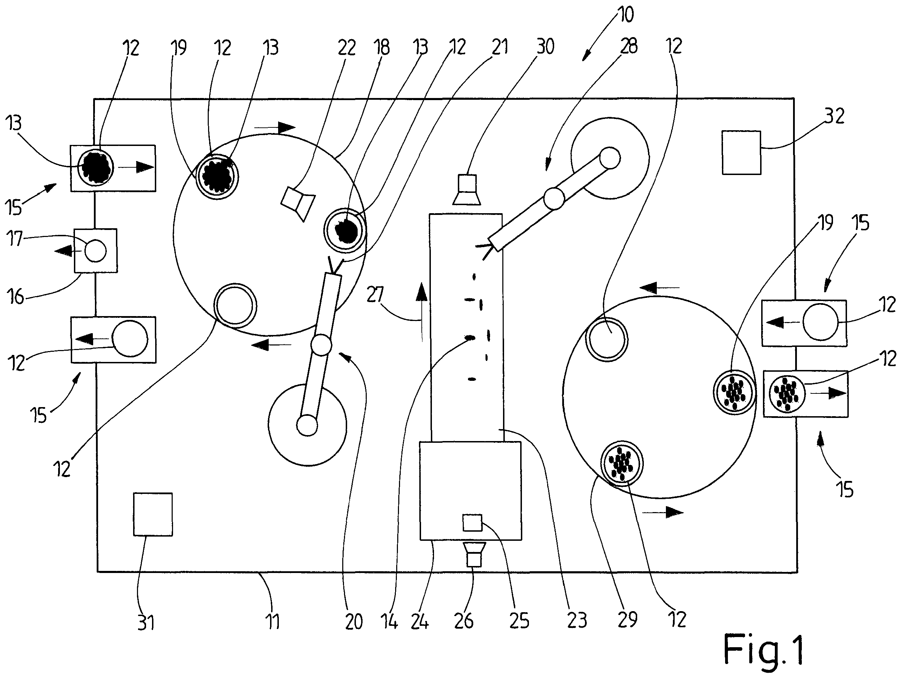

Die in der Fig. 1 stark schematisierte Vorrichtung 10 zum Vermehren von Pflanzen ist im Wesentlichen in einem geschlossenen Raum 11 untergebracht. In diesem Raum 11 befindet sich zumindest während des erfindungsgemäßen Verfahrens zum Vermehren von Pflanzen 13 eine sterile Atmosphäre. Zur Klimatisierung bzw. Belüftung des Raumes 11 befindet sich über diesem Raum 11 ein nicht dargestelltes Entlüftungssystem. Unterhalb des Raumes 11 befindet sich die nicht dargestellte elektronische Steuerung der Vorrichtung 10.

Zum Zuführen von Behältern 12, in denen sowohl die zu vermehrenden Pflanzen 13 als auch deren Klone 14 transportiert werden können, sind dem Raum 11 schleusenartige Zugänge 15 zugeordnet. Diese Zugänge 15 dienen auch dem Ausschleusen der leeren bzw. mit Klonen gefüllten Behälter 12 aus dem Raum 11. Außerdem ist dem Raum 11 ein weiterer schleusenartiger Zugang 16 zugeordnet, um Deckel 17 der Behälter 12 aus dem Raum 11 auszuschleusen.

Die schleusenartigen Zugänge 15, 16 sind besonders geeignet, um die Behälter 12 vollautomatisiert in den Raum 11 ein- bzw. auszuführen, ohne dass ein zu großer Luftaustausch mit der Umgebung der Vorrichtung 10 stattfindet. Dies ist insbesondere für die Aufrechterhaltung der sterilen Atmosphäre in dem Raum 11 notwendig.

Zum Transport der Behälter 12 in der Vorrichtung 10 bzw. in dem Raum 11 sind in dem in der Fig. 1 dargestellten Ausführungsbeispiel zwei drehtellerartige Förderorgane 18, 29 angeordnet. Diese drehtellerartigen Förderorgane 18, 29 lassen sich um eine senkrechte Achse im Uhrzeigersinn drehen und weisen hier jeweils drei Aufnahmen 19 zur Aufnahme der Behälter 12 auf. Diese Aufnahmen 19 weisen einen nicht dargestellten Saugnapf auf, mit dem ein Behälter 12, der sich in der Aufnahme 19 befindet, auf den Förderorganen 18, 29 fixiert werden kann.

Des Weiteren sind den Förderorganen 18, 29 nicht dargestellte, bewegliche Saugnäpfe zugeordnet, mit denen die Deckel 17 der zunächst geschlossenen Behälter 12 abgenommen werden können. Mit diesen Saugnäpfen oder mit weiteren Saugnäpfen können die Behälter 12 auch wieder mit den Deckeln 17 verschlossen werden.

Zur Entnahme der zu vermehrenden Pflanzen 13 aus einem Behälter 12 ist dem Förderorgan 18 ein Greifer 20 zugeordnet. Dieser Greifer 20 besteht im Wesentlichen aus einem mehrachsigen Roboterarm, dem an einem Ende eine sich automatisch öffnende und schließende Pinzette 21 zugeordnet ist. Mit diesem Greifer 20 lassen sich einzelne Pflanzen 13 bzw. Setzlinge aus dem Behälter 12, welcher in eine Aufnahme 19 des Förderorgans 18 positioniert ist, entnehmen. Für das gezielte Entnehmen einer Pflanze 13 durch den Greifer 20 bzw. die Pinzette 21 ist dem Förderorgan 18 eine erste Bilderkennungseinrichtung 22 zugeordnet. Bei dieser Bilderkennungseinrichtung 22 handelt es sich bevorzugt um eine CCD-Kamera, die mit dem elektronischen Steuersystem verbunden ist und bestimmte Formen erkennt und dementsprechend dem Greifer 20 Steuersignale übermittelt, damit dieser punktgenau einzelne Pflanzen 13 ergreifen kann.

Zwischen den Förderorganen 18, 29 ist ein Förderband 23 angeordnet. An einem Ende des Förderbandes 23 befindet sich eine Umhausung 24, in der ein Laser 25 positioniert ist. Mit diesem Laser 25, bei dem es sich beispielsweise um einen CO2-Gaslaser handeln kann, können Pflanzen zerschnitten werden. Damit das Zerschneiden der Pflanzen punktgenau und gesteuert abläuft, ist der Umhausung 24 bzw. dem Laser 25 eine zweite Bilderkennungseinrichtung 26 zugeordnet. Genau wie die erste Bilderkennungseinrichtung 22 ist auch die zweite Bilderkennungseinrichtung 26 mit der elektronischen Steuereinrichtung verbunden. Die Bilderkennungseinrichtung 26 erkennt somit bestimmte Muster, denen entsprechende Schnittlinien zuordbar sind. Diese Informationen über die bestimmten Schnittlinien werden sodann an den Laser 25 übermittelt, damit dieser die einzelnen Pflanzen 13 in Klone 14 zerschneiden kann.

Die einzelnen Klone 14 können sodann über das Förderband 23 in Richtung 27 abtransportiert werden. Einem dem Laser 25 gegenüberliegenden Ende des Förderbandes 23 ist ein weiterer Greifer 28 zugeordnet. Dieser Greifer 28 ist gleichermaßen aufgebaut wie der Greifer 20. Mit diesem Greifer 28 lassen sich die einzelnen Klone 14 der Pflanze 13 von dem Förderband 23 aufnehmen und in sterilen Behältern 12, die sich in den Aufnahmen 19 des Förderorgans 29 befinden, absetzen. Damit der Greifer 28 die einzelnen Klone 14 von dem Förderband 23 erfassen kann, ist dem Förderband 23 eine dritte Bilderkennungseinrichtung 30 zugeordnet. Auch diese Bilderkennungseinrichtung 30 ist genau wie die Bilderkennungseinrichtungen 22 und 26 mit dem elektronischen Steuersystem verbunden und ermittelt bevorzugte Ansatzpunkte für den Greifer 28 an den Klonen 14.

Zum Sterilisieren der Pinzetten 21 der Greifer 20 und 28 ist jedem Greifer 20, 28 eine Einrichtung 31, 32 zum Sterilisieren zugeordnet. In diesen Einrichtungen 31, 32 werden die Pinzetten 21 beispielsweise mit Wärme beaufschlagt, um ggf. Keime abzutöten. Des Weiteren können sich in dem Raum 11 UV-Lampen befinden, um nach der Vermehrung einer Pflanzencharge den gesamten Raum 11 zu bestrahlen und somit zu entkeimen.

Im Folgenden soll anhand der Fig. 1 bis 3 das erfindungsgemäße Verfahren zum Vermehren von Pflanzen beschrieben werden:
Zu Beginn des Verfahrens wird ein Behälter 12, der eine Vielzahl der zu vermehrenden Pflanzen 13 aufweist, durch den Zugang 15 in den Raum 11 eingebracht. Dort wird der Behälter 12 automatisiert an eine Aufnahme 19 des Förderorgans 18 übergeben und von dem Saugnapf in der Aufnahme 19 fixiert. Sodann wird durch einen weiteren beweglichen Saugnapf der Deckel 17 des Behälters 12 entfernt und durch den Zugang 16 aus dem Raum 11 ausgeschleust.

Im Folgenden wird das drehtellerartige Förderorgan 18 mit dem Behälter 12 im Uhrzeigersinn gedreht. In der folgenden Position werden die Pflanzen 13 in den Behältern 12 durch die erste Bilderkennungseinrichtung 22 aufgenommen und über die Steuereinrichtung eine Stelle bzw. ein Blatt einer Pflanze 13 ermittelt, die sich besonders geeignet durch den Greifer 20 ergreifen lässt. Wenn ein derartiges Blatt ermittelt wurde, fährt der Greifer 20 vollautomatisiert genau dieses Blatt an und vereinzelt die Pflanze 13 (Fig. 1). Der Greifer 20 führt sodann die vereinzelte Pflanze 13 in die Umhausung 24.

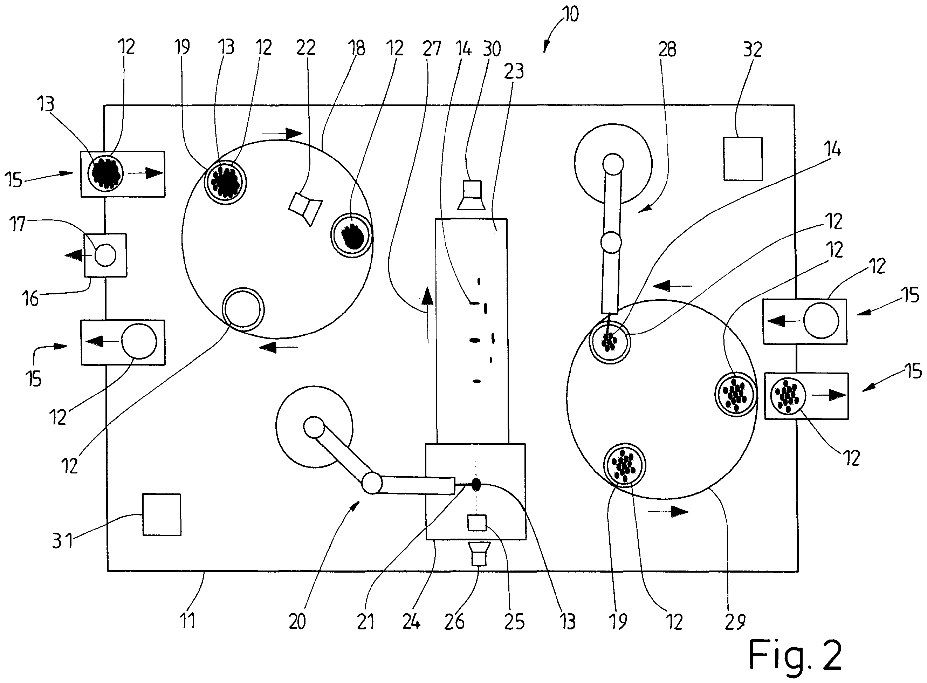

In der Umhausung 24 wird die zu vervielfältigende Pflanze 13 von dem Greifer 20 in eine Position verfahren, welche von einer zweiten Bilderkennungseinrichtung 26 erfasst wird. Diese zweite Bilderkennungseinrichtung 26 ermittelt zusammen mit dem Steuersystem mehrere Schnittlinien, entlang derer die Pflanze 13 zerschnitten werden kann, um Klone 14 der Pflanze 13 zu erstellen. Wenn die Schnittlinien ermittelt wurden, wird die Pflanze 13 durch den Laser 25 in mehrere Klone 14 zerschnitten (Fig. 2). Wenn die Pflanze 13 nicht weiter zerschnitten werden kann, wird diese auf das Band fallen gelassen und der Greifer 20 bewegt sich zurück zum Behälter 12, um eine neue Pflanze 13 zu ergreifen. Dieser Vorgang wird solange fortgesetzt, bis der Behälter 12 keine Pflanzen 13 mehr aufweist. Der leere Behälter 12 wird dann durch das Förderorgan 18 abtransportiert und durch den Zugang 15 aus dem Raum 11 ausgeschleust. Im gleichen Moment wird ein neuer Behälter 12 durch den Zugang 15 in den Raum 11 eingeschleust und ein Behälter bewegt sich in die Position, in der die einzelnen Pflanzen 13 wiederum von dem Greifer 20 ergriffen werden können.

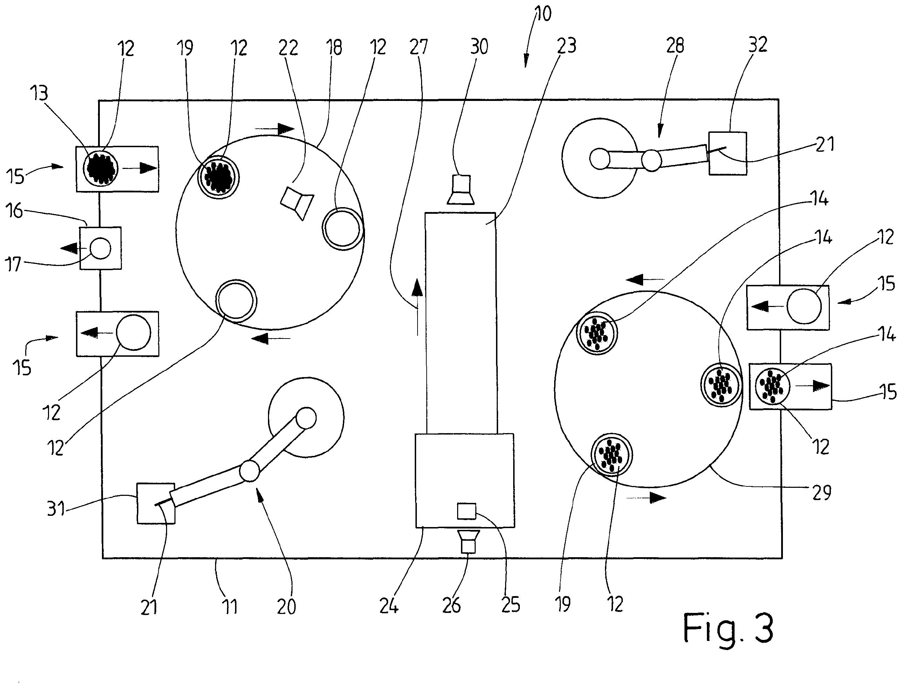

Die von dem Laser 25 zerschnittenen Pflanzen 13 bzw. die Klone werden durch das Förderband 23 in Richtung 27 abtransportiert (Fig. 3). Am Ende des Förderbandes 23 werden die Klone wiederum durch die dritte Bilderkennungseinrichtung 30 erkannt und eine Position ermittelt, an der sich die einzelnen Klone 14 besonders geeignet von dem zweiten Greifer 28 ergreifen lassen. Währenddessen wurde das zweite Förderorgan 29 mit sterilen Behältern 12 bestückt. Diese sterilen Behälter 12 werden ebenfalls durch Zugänge 15 in dem Raum 11 eingeschleust. Auch diese Behälter 12 werden zunächst in den Aufnahmen 19 durch einen Saugnapf fixiert. Der Deckel 17 eines neu eingeführten Behälters 12 wird zunächst durch einen beweglichen Saugnapf abgenommen und sodann an einen weiteren Saugnapf übergeben, welcher einen mit Klonen 14 befüllten Behälter 12 mit dem gleichen Deckel 17 wieder schließt.

Das Förderorgan 29 dreht die Behälter 12 in eine Position, in der der Greifer 28 die einzelnen Klone 14 in den Nährboden der Behälter 12 reindrücken kann. Die Positionierung der einzelnen Klone 14 in den Behältern 12 erfolgt gemäß einem Muster bzw. einer Matrix. Sobald ein Behälter gefüllt ist bzw. alle Plätze einer Matrix besetzt sind, wird, wie soeben beschrieben, der Behälter 12 wieder mit seinem Deckel 17 versehen und durch den Zugang 15 aus dem Raum 11 ausgeschleust.

Sobald eine Charge der zu vermehrenden Pflanzen 13 verarbeitet wurde, werden die Pinzetten 21 der Greifer 20 und 28 zu den Einrichtungen 31, 32 zur Sterilisierung geführt. In dieser Phase kann auch der gesamte Raum 11 durch elektromagnetische Strahlung und/oder Begasung sterilisiert werden. Sobald die Sterilisierung abgeschlossen ist, kann eine neue Charge von Pflanzen 13 vermehrt werden.

### Bezugszeichenliste

- 10: Vorrichtung
- 11: Raum
- 12: Behälter
- 13: Pflanze
- 14: Klon
- 15: Zugang
- 16: Zugang
- 17: Deckel
- 18: Förderorgan
- 19: Aufnahme
- 20: Greifer
- 21: Pinzette
- 22: erste Bilderkennungseinrichtung
- 23: Förderband
- 24: Umhausung
- 25: Laser
- 26: zweite Bilderkennungseinrichtung
- 27: Richtung
- 28: Greifer
- 29: Förderorgan
- 30: dritte Bilderkennungseinrichtung
- 31: Einrichtung
- 32: Einrichtung

## Patentansprüche

1. Verfahren zum Vermehren von Pflanzen (13), wobei die zu vermehrenden Pflanzen (13) zunächst automatisiert durch einen ersten Greifer (20) ergriffen und vereinzelt werden, die einzelnen Pflanzen (13) an dem ersten Greifer (20) hängend gezielt in mehrere Klone (14) zerschnitten und die einzelnen Klone (14) automatisiert durch einen zweiten Greifer (28) für die weitere Verarbeitung abtransportiert werden, wobei durch eine zweite Bilderkennungseinrichtung (26) Schnittlinien an der am ersten Greifer (20) hängenden Pflanze (13) ermittelt werden, entlang derer die Pflanze (13) automatisiert durch ein Messer, einen Laserstrahl (25), einen Wasserstrahl oder einen Plasmastrahl in Klone (14) der Pflanze (13) zerschnitten wird.

2. Verfahren zum Vermehren von Pflanzen (13) nach Anspruch 1, **dadurch gekennzeichnet, dass** die zu vermehrenden Pflanzen (13) in einem zunächst verschlossenen, insbesondere markierten, Behälter (12) in einen geschlossenen, sterilen Raum (11) eingeführt werden und die Behälter (12) in dem Raum (11) automatisiert, insbesondere durch mindestens einen Saugnapf, geöffnet werden.

3. Verfahren zum Vermehren von Pflanzen (13) nach Anspruch 1 oder 2, **dadurch gekennzeichnet, dass** der geöffnete Behälter (12) einer ersten Bilderkennungseinrichtung (22) zugeführt wird und durch die Bilderkennungseinrichtung (22) geeignete Positionen ermittelt werden, an denen die einzelne Pflanzen (13) durch den ersten Greifer (20) nacheinander erfasst und aus dem Behälter (12) entnommen werden.

4. Verfahren zum Vermehren von Pflanzen (13) nach einem der vorhergehenden Ansprüche, **dadurch gekennzeichnet, dass** die Klone (14), vorzugsweise auf einem Förderband (23), zu einer dritten Bilderkennungseinrichtung (30) transportiert werden, von der dritten Bilderkennungseinrichtung (30) Positionen ermittelt werden, an denen die einzelnen Klone (14) durch einen zweiten Greifer (28) ergriffen und in einem Behälter (12), vorzugsweise gemäß einem Raster in dem Behälter (12), abgesetzt werden.

5. Verfahren zum Vermehren von Pflanzen (13) nach einem der vorhergehenden Ansprüche, **dadurch gekennzeichnet, dass** der eine Nährlösung enthaltene Behälter (12) vor der Aufnahme der Klone (14) in dem sterilen Raum (11) geöffnet und nach der Aufnahme der Klone (14) in dem Raum (11) wieder geschlossen wird, bevor der Behälter (12) aus dem Raum (11) abtransportiert wird.

6. Verfahren zum Vermehren von Pflanzen (13) nach einem der vorhergehenden Ansprüche, **dadurch gekennzeichnet, dass** der Raum (11) und/oder die Greifer (20, 28) regelmäßig, insbesondere nach dem Durchgang einer zu vermehrenden Pflanzencharge, sterilisiert werden.

7. Vorrichtung (10) zum Vermehren von Pflanzen (13) mit mindestens einem ersten Greifer (20) zum gezielten Ergreifen und Vereinzeln einer zu vermehrenden Pflanze (13), mit mindestens einer Einrichtung zum automatisierten Zerschneiden der Pflanze (13) in mehrere Klone (14), mit mindestens einem zweiten Greifer (28) zum automatisierten Abtransport einzelner Klone (14) und einem Raum (11), in dem die Greifer (20, 28) und die mindestens eine Einrichtung zum Zerschneiden angeordnet sind, wobei die Einrichtung zum automatisierten Zerschneiden der an dem ersten Greifer hängenden Pflanze (13) ein Laser (25), ein Plasmagenerator, ein Messer oder ein Wasserschneider ist, der eine Bilderkennungseinrichtung (26) zugeordnet ist zur Ermittlung geeigneter Schnittlinien an der an dem ersten Greifer hängenden Pflanze (13), wobei der Einrichtung zum automatisierten Zerschneiden der Pflanze (13) ein Förderband (23) zum Abfördern der Klone (14) zugeordnet ist.

8. Vorrichtung (10) zum Vermehren von Pflanzen (13) nach Anspruch 7, **dadurch gekennzeichnet, dass** der Raum (11) ein steriler Reinraum ist mit mehreren Zugängen (15, 16), insbesondere Schleusen, für das Einschleusen und Ausschleusen von Behältern (12) mit den zu vermehrenden Pflanzen (13) und Klonen (14), wobei dem Raum (11), insbesondere den Zugängen (15, 16) des Raumes (11), mindestens zwei Förderorgane (18, 29), vorzugsweise Drehteller, für die Behälter (12) zugeordnet sind, mit denen die Behälter (12) automatisiert in vorbestimmte Positionen verfahrbar sind.

9. Vorrichtung (10) zum Vermehren von Pflanzen (13) nach Anspruch 7 oder 8, **dadurch gekennzeichnet, dass** den Förderorganen (18, 29) mindestens eine Einrichtung, insbesondere mindestens ein Saugnapf, zum automatisierten Öffnen und/oder Schließen der Behälter (12) zugeordnet ist.

10. Vorrichtung (10) zum Vermehren von Pflanzen (13) nach einem der Ansprüche 7 bis 9, **dadurch gekennzeichnet, dass** mindestens einem Förderorgan (18, 29), vorzugsweise beiden Förderorganen (18, 29) und/oder einem Förderband (23), jeweils eine Bilderkennungseinrichtung (22, 30) zugeordnet ist zur Ermittlung einer geeigneten Position, an der eine Pflanze (13) und/oder ein Klon (14) durch den ersten und/oder den zweiten Greifer (20, 28), insbesondere durch eine an einem mehrachsigen Roboterarm angeordnete Pinzette (21), automatisiert greifbar ist.

## Claims

1. A method for propagating plants (13), wherein the plants (13) to be propagated are firstly automatically grasped by a first gripper (20) and separated, the individual plants (13), hanging on the first gripper (20), are systematically cut into a plurality of clones (14), and the individual clones (14) are automatically transported away by a second gripper (28) for further processing, wherein cutting lines on the plant (13) hanging on the first gripper (20) are determined by a second image recognition unit (26), along which cutting lines the plant (13) is automatically cut by a blade, a laser beam (25), a waterjet, or a plasma jet into clones (14) of the plant (13).

2. The method for propagating plants (13) as claimed in claim 1, **characterized in that** the plants (13) to be propagated are introduced in an initially closed, in particular marked container (12) into a closed, sterile room (11) and the containers (12) are automatically opened in the room (11), in particular by at least one suction cup.

3. The method for propagating plants (13) as claimed in claim 1 or 2, **characterized in that** the open container (12) is supplied to a first image recognition unit (22) and suitable positions are determined by the image recognition unit (22), at which the individual plants (13) are successively grasped by the first gripper (20) and removed from the container (12).

4. The method for propagating plants (13) as claimed in any one of the preceding claims, **characterized in that** the clones (14) are transported, preferably on a conveyor belt (23), to a third image recognition unit (30), positions are determined by the third image recognition unit (30), at which the individual clones (14) are grasped by a second gripper (28) and placed in a container (12), preferably according to a grid in the container (12).

5. The method for propagating plants (13) as claimed in any one of the preceding claims, **characterized in that** the container (12) containing a nutrient solution is opened in the sterile room (11) before the accommodation of the clones (14) and is closed again in the room (11) after the accommodation of the clones (14), before the container (12) is transported away out of the room (11).

6. The method for propagating plants (13) as claimed in any one of the preceding claims, **characterized in that** the room (11) and/or the grippers (20, 28) are sterilized regularly, in particular after the passage of a plant batch to be propagated.

7. A device (10) for propagating plants (13) having at least one first gripper (20) for the systematic grasping and separation of a plant (13) to be propagated, having at least one apparatus for automatically cutting the plant (13) into multiple clones (14), having at least one second gripper (28) for automatically transporting away individual clones (14), and a room (11), in which the grippers (20, 28) and the at least one apparatus for cutting are arranged, wherein the apparatus for automatically cutting the plant (13) hanging on the first gripper is a laser (25), a plasma generator, a blade, or a water cutter, with which an image recognition unit (26) is associated for determining suitable cutting lines on the plant (13) hanging on the first gripper, wherein a conveyor belt (23) for conveying away the clones (14) is associated with the apparatus for automatically cutting the plant (13).

8. The device (10) for propagating plants (13) as claimed in claim 7, **characterized in that** the room (11) is a sterile clean room having multiple accesses (15, 16), in particular airlocks, for the inward transfer and outward transfer of containers (12) having the plants (13) to be propagated and the clones (14), wherein at least two conveyor elements (18, 29), preferably turntables, for the containers (12) are associated with the room (11), in particular the accesses (15, 16) of the room (11), using which conveyor elements the containers (12) are automatically movable into predetermined positions.

9. The device (10) for propagating plants (13) as claimed in claim 7 or 8, **characterized in that** at least one apparatus, in particular at least one suction cup, for automatically opening and/or closing the containers (12) is associated with the conveyor elements (18, 29).

10. The device (10) for propagating plants (13) as claimed in any one of claims 7 to 9, **characterized in that** an image recognition unit (22, 30) is associated in each case with at least one conveyor element (18, 29), preferably both conveyor elements (18, 29) and/or a conveyor belt (23), for determining a suitable position, at which a plant (13) and/or a clone (14) can be automatically grasped by the first and/or the second gripper (20, 28), in particular by tweezers (21) arranged on a multiaxis robot arm.

## Revendications

1. Procédé de reproduction de plantes (13), dans lequel les plantes à reproduire (13) sont d'abord attrapées et isolées de façon automatisée par une première griffe (20), les plantes individuelles (13) sont découpées en plusieurs clones (14) de façon ciblée après avoir été accrochées au niveau de la première griffe (20) et les clones individuels (14) sont évacués de façon automatisée à l'aide d'une deuxième griffe (28) pour leur traitement ultérieur, dans lequel des lignes de découpe sont déterminées au niveau de la plante (13), accrochée au niveau de la première griffe (20), par le biais d'un deuxième dispositif de reconnaissance d'image (26), la plante (13) étant découpée de façon automatisée le long desdites lignes en clones (14) de la plante (13) au moyen d'un couteau, d'un rayon laser (25), d'un jet d'eau ou d'un rayon plasma.

2. Procédé de reproduction de plantes (13) selon la revendication 1, **caractérisé en ce que** les plantes à reproduire (13) sont introduites dans un récipient (12) d'abord obstrué, notamment marqué, dans une chambre stérile fermée (11), et les récipients (12) sont ouverts de façon automatisée dans la chambre (11), notamment au moyen d'au moins une ventouse.

3. Procédé de reproduction de plantes (13) selon la revendication 1 ou 2, **caractérisé en ce que** le récipient ouvert (12) est amené à un premier dispositif de reconnaissance d'image (22) et que des positions adaptées sont déterminées par le dispositif de reconnaissance d'image (22) au niveau desquelles les plantes individuelles (13) seront détectées à la suite les unes à la suite des autres par la première griffe (20) puis retirées du récipient (12).

4. Procédé de reproduction de plantes (13) selon l'une quelconque des revendications précédentes, **caractérisé en ce que** les clones (14) sont transportés jusqu'à un troisième dispositif de reconnaissance d'image (30), de préférence sur un tapis roulant (23), que les positions auxquelles les clones individuels (14) sont agrippés par une deuxième griffe (28) sont déterminées par le troisième dispositif de reconnaissance d'image (30) et sont décalées dans un récipient (12), de préférence en fonction d'un quadrillage prévu dans le récipient (12).

5. Procédé de reproduction de plantes (13) selon l'une quelconque des revendications précédentes, **caractérisé en ce que** le récipient (12) contenant une solution nutritive est ouvert avant la réception des clones (14) dans la chambre stérile (11) et est à nouveau refermé après la réception des clones (14) dans la chambre (11) avant que le récipient (12) soit évacué de la chambre (11) .

6. Procédé de reproduction de plantes (13) selon l'une quelconque des revendications précédentes, **caractérisé en ce que** la chambre (11) et/ou les griffes (20, 28) sont régulièrement stérilisées, notamment après le passage d'un lot de plantes reproduites.

7. Dispositif (10) de reproduction de plantes (13) avec au moins une première griffe (20) permettant d'attraper de façon ciblée une plante à reproduire (13), avec au moins un dispositif de découpage automatisé de la plante (13) en plusieurs clones (14), avec au moins une deuxième griffe (28) permettant l'évacuation automatisée des clones (14) individuels et avec une chambre (11) dans laquelle la griffe (20, 28) et l'au moins un dispositif de découpe sont disposés, dans lequel le dispositif de découpe automatisé de la plante (13) accrochée au niveau de la première griffe est un laser (25), un générateur plasma, un couteau ou un système de découpe par l'eau qui est associé à un dispositif de reconnaissance d'image (26) pour déterminer les lignes de découpe adaptées au niveau de la plante (13) accrochée à la première griffe, dans lequel un tapis roulant (23) est associé au dispositif de découpe automatisée de la plante (13) en vue d'évacuer les clones (14).

8. Dispositif (10) de reproduction de plantes (13) selon la revendication 7, **caractérisé en ce que** la chambre (11) est une sale blanche stérile avec plusieurs accès (15, 16), notamment des sas permettant d'entrer et de sortir de façon contrôlée les récipients (12) avec les plantes à reproduire (13) et les clones (14), dans lequel au moins deux organes de transport (18, 29), de préférence des plateaux tournants prévus pour les récipients (12), sont associés à la chambre (11), notamment aux accès (15, 16) de la chambre (11) au travers desquels les récipients (12) sont amenés de façon automatisée dans des positions prédéfinies.

9. Dispositif (10) de reproduction de plantes (13) selon la revendication 7 ou 8, **caractérisé en ce qu'**au moins un dispositif, notamment au moins une ventouse, est associé aux organes de transport (18, 29) en vue de réaliser l'ouverture et/ou la fermeture automatisées des récipients (12).

10. Dispositif (10) de reproduction de plantes (13) selon l'une quelconque des revendications 7 à 9, caractérisé en ce respectivement un dispositif de reconnaissance d'image (22, 30) est associé à au moins un organe de transport (18, 29), de préférence aux deux organes de transport (18, 29) et/ou à un tapis roulant (23), en vue de déterminer une position adaptée au niveau de laquelle une plante (13) et/ou un clone (14) est attrapé de façon automatisée par la première et/ou la deuxième griffe (20, 28), notamment au travers d'une pincette (21) disposée au niveau de bras de robot à axes multiples.
